# EUROPEAN PATENT APPLICATION

(11) **EP 3 892 187 A1**
(43) Date of publication of application: **13.10.2021**
(21) Application number: 20020175.4
(22) Date of filing: 12.04.2020
(51) Int. Cl.: A61B 5/01, A61L 2/10, A61L 2/16, A61L 2/24

(54) **SANITARY CONTROL ACCESS SYSTEM FOR EPIDEMIC PREVENTION**

(71) Applicant: Abomailek Rubio, Basel Carlos, 08227 Terrassa, Barcelona (ES)
(72) Inventor: Abomailek Rubio, Basel Carlos, 08227 Terrassa, Barcelona (ES); Abomailek Thabet, Medhat, 08227 Terrassa, Barcelona (ES)

(57) **Abstract**

Problem statement: To safely control access to highly concurred places in optimal hygienic and health status of individuals or goods for epidemic prevention. Solution: In the present invention FIG.1 , a sanitary control access system (1) for epidemic prevention is installed at the entrances of highly-concurred buildings or, generally, facilities. The purpose of such is to control the access of people and goods under the premises of ensuring the hygienic conditions. To perform an evaluation of the state of health of users based on their body-temperature, to determine whether is safe to let it enter or in case to provide, personal protection equipment or health assistant. Furthermore, the system is aimed to provide automatic assisted sanitizing by combining a nebulization of sanitizing agent through the body of the user or the goods surface, plus compressed air cleansing and further a disinfection treatment with Far ultraviolet C light.

## Description

### OBJECT OF THE INVENTION

The field of the invention relates to an automatic control access system enhanced with an automatic hygiene system devoted to sanitize users and goods that must cross highly concurred places before accessing to such places and to prevent and redirect users in case they are detected to be possibly carrying contagious diseases.

An object of the invention is to provide a system and method capable of providing automated access control to highly concurred places making a focus in applying a full sanitizing method to the system user.

An object of the invention is to provide a system and method capable of offering a safe, fast and automatic sanitizing of individuals and goods.

Another object of the invention is to avoid high spread of highly contagious pathogens by means of dramatically reducing the viral charge in individuals entering or exiting highly concurred places.

Another object of the invention is to diminish the intensity of infectious diseases by decreasing the viral charge of infected individuals by enforcing systematic prophylaxis at the entrance of highly concurred spaces.

Another object of the invention is to evaluate health state of users by means of body-temperature measurement in order to avoid entrance of such to the facility and to redirect them to obtain health-aid.

### BACKGROUND OF THE INVENTION

A significant public health problem: The expansion of SARS-CoV-2 virus, and the subsequent spread of COVID-19 has put into resource stress the health systems of several countries around the world. Specifically, scarcity of personal protective material such as hand sanitizer, FFP3 masks, medical gowns and others aimed to protect individuals against disease spread through contact and air, has been at the center of the pandemic worldwide spread.

Furthermore, the facility and ubiquity of communications throughout the globe has made possible the spread of the virus not only due to direct travelling of infected individuals to third countries. But, also, due to indirect interaction in high transit facilities such as, -but not only- airports, train stations, cruises, stadiums, museums, office buildings and others.

Nowadays, none of the aforementioned places and extensively any high concurrence place is able to ensure proper hygiene of their users. Although in some, hand sanitizers are offered, no system has a holistic view on the topic. Thus, none of these places are able to ensure the full-body sanitizing of all of their users and the goods they transport.

Awareness of the necessity of ubiquitous sanitizing systems will dramatically grow during and after this crisis. Therefore, the present invention proposes an invention able to provide both automatic access control and full-body sanitizing, altogether in an easy deployable system.

The aim of that is to decrease virus distribution and virial intensity disease based on three factors.

The first is that eliminating virus carried through the body diminishes dramatically the possibilities of personal infection and infection to thirds.

The second is that having means for detecting possibly infected users by means of temperature measure permits to provide personal protection equipment to users, to redirect them through specific paths or to avoid allowance of entrance to the facility.

The third is that decreasing the overall viral charge is straightly related with less severe diseases. Less severe infections may benefit the dimensioning of health facilities as the ratio of use of intensive care units per infected people will drop accordingly.

### DESCRIPTION OF INVENTION

The present invention overcomes the above mentioned problems by providing a system that enables fast and automatic access control for persons and goods, full-body sanitizing for persons, and precise control of body-temperature of persons.

The system comprises a cabin, module, frame or generally an embodiment that includes an electronic system to detect the entrance of the user, recognizes its position and face status, measures its body-temperature and gives instructions to the user to proceed to an automatic and safe sanitizing.

The system is able to determine whether a user, a good or both at the same time access to the main invention embodiment. And so, it is able to apply the sanitizing process safely to both.

The sanitary control access system according the present invention may detect the entering user temperature and accordingly allow the user to enter or to propose to use personal protective equipment or to be redirected to the health service.

The sanitary control access system according the present invention may detect the incoming user and its face. Following, it may indicate safety instructions to guide the user such as -but not limited-"Stop", "Close the eyes", "expose your hands palm", "Please, put on the available disposable globes, mask and gown", "Please continue".

In the aforementioned embodiment, the system also comprises two sanitizing systems. The first, a liquid solution nebulizer and a set of supply hoses aimed to dispense disinfecting solution or compressed air on the user. The second, a Far Ultra Violet C lamp or set of lamps aimed to reinforce the virus elimination at the user surface and the surfaces of the cabin itself.

The sanitary control access system for epidemic prevention according the present invention may generate Far Ultra Violet C light comprising a wavelength between (207-222 nm) to perform harmless, disinfection.

The sanitary control access system for epidemic prevention system according the present invention may nebulize a disinfecting solution through the user body, clothes, accessories, goods and others, without requiring further manual substance distribution.

The sanitary control access system for epidemic prevention system according the present invention may include a compressed air system for mechanically removal of pathogen's on either the surfaces of users or goods and a floor able to pick and deactivate such pathogens.

The embodiment includes a deposit for the storing of chemical disinfecting products and a housing for the control and power units necessary for the face-detecting electronics and the Far Ultra Violet C lamps.

In the event of being the user unable to understand the instructions, the sanitary control access system for epidemic prevention may have a set of actuators that could be operated from outside to perform the sanitizing procedure in a human assisted way.

Various objects, features, aspects and advantages of the present invention will become more apparent from the following detailed description of preferred embodiments of invention, along with the accompanying drawings in which like numerals represent like components.

### BRIEF DESCRIPTION OF DRAWINGS

FIG.1 is a schematic of the sanitary access control system for epidemic prevention in use.
FIG 2. Depicts a proposal of integration of the personal disinfection system in the entrance of a building.

### DESCRIPTION OF PREFERRED EMBODIMENTS

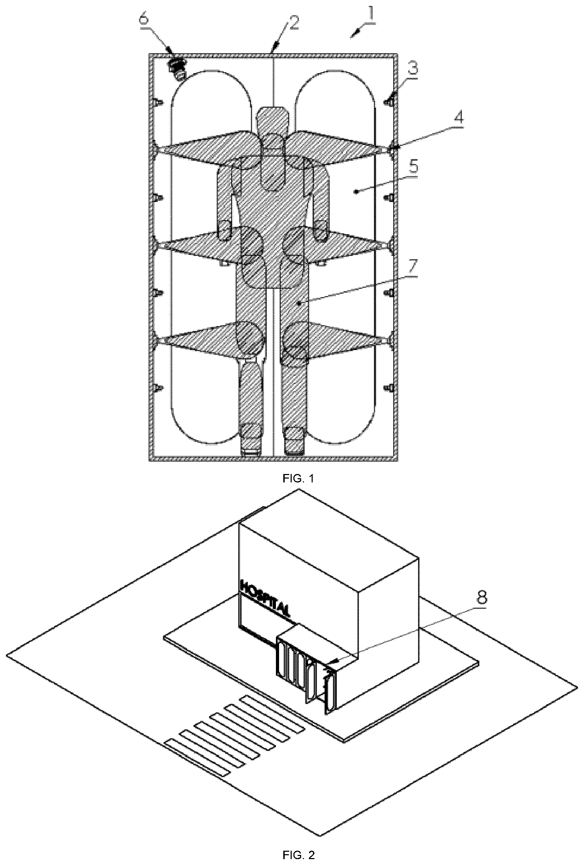

With reference to FIG. 1, a preferred sanitary access control system for epidemic prevention fully assembled **1.** The system comprises. FIG. 2, the sanitary access control system for epidemic prevention **1** appears integrated in a building entrance **8**, and in a bundle of three preferred sanitary access control systems for epidemic prevention.

The system **1** includes an enclosure **2** with two doors **5** for permitting the circulation of the users **7** whilst applying the sanitizing procedure. The system **1** includes, as well, the necessary deposits, power transformer modules, piping, electronics and electrical wiring necessary to provide functionality to the system. In order to apply the two proposed methodologies for user **1** and belongings sanitizing. A set of Far-UVC lamp **3** and a of sanitizing solution dispenser nozzles **4** are place all along the lateral walls of the enclosure **1.** Furthermore, in order to measure body-temperature and to perform face-detection methodologies to ensure safety during the sanitizing a camera **6** is placed on top of the enclosure **1.** Finally, a system for providing instructions to user **7** for proper and safe sanitizing is included in the embodiment.

## Claims

1. System (1) for automatic sanitary control of access and personal disinfection for epidemic prevention, comprising:
- an embodiment (2) for a user (3) and goods (10) to be disinfected,
- a contact-less body-temperature measure system (4) to determine the accessing user state of health.
- a face detection system (5) **characterized in that** the face detection system (5) is configured to:
a) detect the facial data (6) of users by means of a system able to capture its image (7).
b) process the facial data (6) to determine what is the status of the user.
c) give instructions to the user by means of an audio or visual system (8) to actuate accordingly to the needs of the disinfection.
- a Far Ultraviolet C lighting system (9) able to disinfect the surfaces and the air surrounding the user (3) composed by:
d) a set of Far Ultraviolet C lamps (9) placed inside the embodiment (2) to fully irradiate a user (5) surface, its surroundings and goods (10) within the embodiment (2).
e) the power system (11) to energize the Far Ultraviolet C lamps (9)
- a Nebulizing system (12) consisting in:
f) a deposit (13) of sanitizing agent (14).
g) a set of Nebulizing nozzles (16) placed across the embodiment (2) to properly irrigate the user (3) surface and the surface of goods (10) within the embodiment (2) with the sanitizing agent (14) in order to reinforce user sanitation performed with the Far Ultraviolet C lighting system (9)
h) a set of hoses (15) for distributing the sanitizing agent (14) from the deposit (13) to the Nebulizing nozzles (16).
- a pathogen mechanical removal system (19) consisting in:
i) a deposit (20) and an air-pump (21)
g) a set of compressed-air nozzles (22) placed across the embodiment (2) to properly access the user (3) surface and the surface of goods (10) within the embodiment (2).
k) a set of compressed-air hoses (23) for distributing the compressed-air from the deposit (20) to the nozzles (22)
- a pair of access doors (17) aimed to effectively control the access from a place to the embodiment (2) and from the embodiment to a different place.
- the control system (18) and power system (11) needed to energize and operate the full system (1), and evaluate whether the user or goods are sanitized and decide if access is permitted, restricted or permitted following some requirements.

2. System (1) according to claim 1, wherein the contact-less body-temperature measurement system (4) comprising a mean to measure the temperature of a user (3) accessing the embodiment (2).

3. System (1) according to claim 1, further comprising: a face detection system (5) comprising a mean to capture the face image or the face expression, generally the facial data (6) of the user (3).

4. System (1) according to claim 1, 4, 5, wherein the face detection system (5) is able to process the facial data (6) in order to provide instructions to the user (3) in order to safely perform user (3) disinfection.

5. System (1) according to claim 1, wherein Far Ultraviolet C lighting system (9) is able to sanitize the user (3) or goods (10) by means of harmless Far Ultraviolet C light-waves.

6. System (1) according to claim 1 wherein Nebulizing system (12) is able to sanitize the user (3) or goods (10) by means of applying a sanitizing agent (14).

7. System (1) according to claim 1 wherein pathogen mechanical removal system (19) is able to remove surficial debris and pathogens from the user (3) or goods (10) by means of blowing air at an a pressure higher than the atmospheric and to manage such pathogens at the embodiment (2) floor.

8. System (1) according to claim 1, 2, 3, 4, 5, 6 wherein the pair of accessing doors (17) automatically allow or restrict the access from the former place to the later place based on the evaluation made by the control system (18) of the state of health of user (3), and the status of sanitizing of user (3) and goods (10) after applying the sanitizing processes.

9. System (1) according to claim 2,3,5,6 wherein the contact-less body temperature measurement system (5), the face detection systems (5), the Nebulizing system (12), the Far Ultraviolet C lighting system (9) are automatically activated when the user (3) or goods (10) access the embodiment.

10. System (1) according to claim 1, wherein alternative manual operation of the control system (18) is introduced to allow the performance of the sanitation process either if a failure in the electronics occur or the person cannot understand or interact with the machine due to low, old age or manifest disability.

11. Method according to claims 1, 4, 6, 8 further comprising:
l) determine whether is accessing to the embodiment (2) a user (3), a good (10) or simultaneously a user (3) and a good (10).
m) if a user (3) or a user (3) and a good (10) access the embodiment (2): obtaining a measure of the body-temperature by means of the contact-less body temperature measure system (4) to determine the user (3) state of health by comparison with average febrile status temperature.
n) if a user (3) or a user (3) and a good (10) access the embodiment (2): obtaining a plurality of facial data (6) representative of the characteristics and the state of user (3) face.
o) if a user (3) or a user (3) and a good (10) access the embodiment (2): introducing the facial data (6) in a previously trained algorithm to online determining whether the user (3) is following the instructions of use of the system (1).
p) if a user (3) or a user (3) and a good (10) access the embodiment (2): a series of audio or visual actuators to provide use instructions to the system (1) user (3) throughout the sanitizing process.
q) perform the sanitizing process by starting the nebulizing system (12) and starting the Far Ultra-violet C system (9).
r) if a good (10) alone access the embodiment: perform the sanitizing process described in n) directly.
s) a system for evaluating the result of the state-of-health of the user (3) and the sanitizing process of the user (3) and goods (10) found within the embodiment (1) and thus, able to determine whether access is allowed at all, allowed under any conditions, or not allowed. If access is allowed, access doors (17) open.

12. Computer program comprising program code means adapted to perform the method steps of claim 10 when said program is run on a general purpose processor, digital signal processor, an FPGA, an ASIC, a microprocessor, a microcontroller, or any other form of programmable hardware.
